# EUROPEAN PATENT APPLICATION

(11) **EP 2 036 534 A1**
(43) Date of publication of application: **18.03.2009**
(21) Application number: 08013907.4
(22) Date of filing: 04.08.2008
(51) Int. Cl.: A61K 8/03, A61K 8/37, A61Q 5/12

(54) **Multi phase composition for conditioning hair**

(30) Priority: 07.08.2007 EP 07015476
(71) Applicant: KPSS-Kao Professional Salon Services GmbH, 64297 Darmstadt (DE)
(72) Inventor: Hoffmann, Martin, 64673 Zwingenberg (DE); Molenda, Michael, 60487 Frankfurt (DE); Grit, Mustafa, Dr., 64579 Gernsheim (DE)
(74) Representative: Grit, Mustafa

(57) **Abstract**

The inventors of the present invention have surprisingly found out that mixing an additional component prior to application which is preferably an emulsion to already existing two or more phase composition comprising an oil phase and a water phase wherein two or more phases are optically separated at zero shear rate and becomes homogeneous upon shaking and returns again to optically separated two or more phases upon release of agitation and mixes to a homogeneous composition when all phases are mixed improves hair shine, elasticity, manageability and combability and adds volume and body when especially a compound described below is included into the composition. It should be noted that after mixing all phase the composition is used immediately or in a short period of time so that the formed composition by mixing all phases does not have to have long term stability. It is enough when it is homogeneous during application onto hair.

Accordingly, the object of the present invention is a multiphase composition for hair comprising
a- 5 to 30%, by weight oil phase, calculated to total composition, comprising at least one volatile oil,
b- 50 to 75%, by weight, aqueous phase, calculated to total composition, and
c- 5 to 45% by weight calculated to total composition an additional composition preferably an emulsion comprising at least one oil and at least one emulsifier in an aqueous medium,

wherein a compound selected from compounds according to general formula 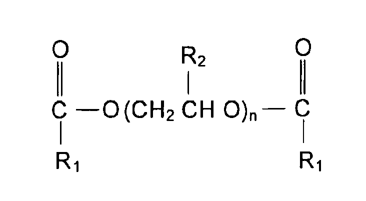 wherein R₁ is a phenyl and R₂ is H or a C 1 to C4 alkyl and n is a number between 1 and 5 and compounds of dipropylene glycol dibenzoate and trimethylpentanediyl dibenzoate is included into one of the phases except aqueous phase.

## Description

Present invention is related to a multi phase, especially three phase, composition for conditioning hair wherein two o the phases are stored in the same package together and they are optically separated and the third phase is kept separate until use either in the same package in a separate compartment or in a separate vessel, and all three phases are mixed to a homogeneous composition prior to application onto hair.

Conditioning compositions have been known for many years. Among them it is possible to find products on hair care market for conditioning hair including two separated phases. Such products have been attracting consumers' attention because of their appearance. Since these products are designed mainly for leave in usage, hair is not rinsed off after application of the product, achieving high care level and combining the conditioning benefit for example with additional one or more benefits such as better combability and improved bodifying effects is found especially beneficial.

Two phase compositions on the market deliver good conditioning levels. However there is still need for improvements especially in hair shine, and, further, conditioning effects of such compositions.

WO 02/060397 discloses dual phase conditioning and styling composition for heat styling hair. According to the document compositions comprise an oil phase comprising volatile oil and an aqueous alcoholic phase comprising styling polymer, an emulsifier and a salt.

EP 996 408 discloses dual phased compositions for make up cleansing wherein a polyvinylpyrrolidone copolymer is used as a demixing agent. The documents does not address at all hair care application of such composition.

Three phase compositions in the form of three optically separated phases in the same vessel are disclosed in EP 1593364 A1. Furthermore, three phase compositions as a bath additive and as a skin care additive are disclosed in EP 882 442 A1 and GB 2259015 A1, respectively.

The inventors of the present invention have surprisingly found out that mixing an additional component prior to application which is preferably an emulsion to already existing two or more phase composition comprising an oil phase and a water phase
wherein two or more phases are optically separated at zero shear rate and becomes homogeneous upon shaking and returns again to optically separated two or more phases upon release of agitation and mixes to a homogeneous composition when all phases are mixed improves hair shine, elasticity, manageability and combability and adds volume and body when especially a compound described below is included into the composition. It should be noted that after mixing all phase the composition is used immediately or in a short period of time so that the formed composition by mixing all phases does not have to have long term stability. It is enough when it is homogeneous during application onto hair.

Accordingly, the object of the present invention is a multiphase composition for hair comprising
a- 5 to 30%, by weight oil phase, calculated to total composition, comprising at least one volatile oil,
b- 50 to 75%, by weight, aqueous phase, calculated to total composition, and
c- 5 to 45% by weight calculated to total composition an additional composition preferably an emulsion comprising at least one oil and at least one emulsifier in an aqueous medium,
wherein a compound selected from compounds according to general formula 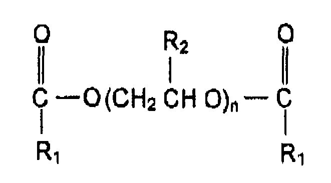 wherein R₁ is a phenyl and R₂ is H or a C1 to C4 alkyl and n is a number between 1 and 5 and compounds of dipropylene glycol dibenzoate and trimethylpentanediyl dibenzoate is included into one of the phases except aqueous phase.

In the preferred form of the invention the phases a an b mentioned above are stored in the same vessel as optically separated two or more phases and the additional composition c is kept separate until use in a different packaging and all phases are mixed together into a homogeneous composition in another vessel prior to application onto hair.

In another preferred embodiment of the present invention, the phases a an b mentioned above are stored in the same vessel as optically separated two or more phases and additional composition c is kept separate until use in the same packaging in a separate compartment and all phases are mixed together into a homogeneous composition in the same vessel prior to application onto hair.

Packaging material is available for storing the above disclosed composition in the same vessel. Such vessels include a main vessel where the phases a and b of the inventive composition can be stored as optically separated two or more phases and includes a separate compartment usually above the main vessel wherein the additional composition can be stored separated from the others until use. Such vessel has a cap which can be turned into a direction which results in loosing the separate compartment on top of the vessel and dropping into the main vessel and by shaking it allows mixing the components into a homogeneous mixture. Such material are commercially available on the market and there are products sold on the market from the applicant. There may certainly be other packaging materials available which allow storing the composition of the present invention,

Non-limiting examples to the compounds of the above formula are diethyleneglycol dibenzoate, propylene glycol dibenzoate and triethylene glycol dibenzoate.

Preferred are dipropylene glycol dibenzoate, diethyleneglycol dibenzoate, propylene glycol dibenzoate and triethylene glycol dibenzoate. More preferred are dipropylene glycol dibenzoate, diethyleneglycol dibenzoate and propylene glycol dibenzoate. Most preferred is the compound propylene glycol dibenzoate which is available from Inolex Chemical Company under the trade name LexFeel Shine.

Concentration of the compounds mentioned above is in the range of 0.1 to 20% preferably 0.1 to 15%, more preferably 0.25 to 10% and most preferably 0.5 to 5% by weight calculated to the total composition.

Compositions of the present invention comprises oil phase (phase a) at a concentration of 5 to 30%, preferably 7.5 to 25% more preferably 10 to 20% by weight calculated to total composition.

Oil phase comprises 70 to 99.9%, preferably 80 to 99.9% and more preferably 90 to 99.9% by weight calculated to the content of oil phase at least one volatile oil. Suitable volatile oils are silicones such as dimethicone and cyclomethicone and mixtures thereof, and volatile hydrocarbons such as isododecane, isohexadecane and isoprafin. The most preferred are silicones and from those dimethicone and cylomethicone and mixtures thereof.

Suitable volatile dimethicones are the ones with a viscosity of less than 5 mm²/s. The most preferred are dimethicone with a viscosity of 1 mm²/s and 0.65 mm²/s which are available from Dow Corning with the trade name DC 200 Fluid.

### Suitable volatile cyclomethicones are according to general formula

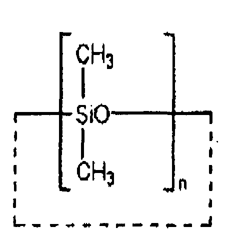

where n is a number between 3 and 7. Preferred are cyclopentasiloxanes known with the trade name for example Dow Corning 245. Non-volatile silicones may also be incorporated into the compositions of the present invention at a low concentration, i.e. lower than 1 % by weight calculated to the content of oil phase. Suitable ones are arylated silicones such as phenyltrimethicone known with the trade name Dow Corning 556, and phenyl methicone, diphenyl dimethicone, diphenylsiloxy phenyl trimethicone, tetramethyl tetraphenyl trisiloxane, triphenyl trimethicone, tetramethly tetraphenyl trisiloxane and trimethyl pentaphenyl trisiloxane and dimethicones with higher viscosity.

In addition, natural oils such as olive oil, almond oil, avocado oil, wheatgerm oil, ricinus oil can be incorporated into the oil phase. Concentration of natural oils is below 1 %, preferably below 0.5% by weight calculated to the content of the oil phase. Solubility of the natural oil in used volatile oil should be examined in selecting natural oil. As a rule selected natural oil should be soluble in the volatile oil used in the composition.

Compositions of the present invention comprise aqueous phase at a concentration of 50 to 95%, preferably 60 to 90% more preferably 70 to 85% by weight calculated to total composition.

Aqueous phase of the compositions of the present invention comprises 10 to 40%, preferably 15 to 35%, more preferably 20 to 30% by weight calculate to the content of aqueous phase at least one water miscible organic solvent-. With the term "water miscible" it is meant that the organic solvent is miscible with water at any ratio. Suitable ones are C₂ - C₄ monohydric alcohols such as ethanol, propanol, isopropanol, butanol and isobutanol, benzyl alcohol and benzyloxyethanol and their mixtures. Most preferred are ethanol and isopropanol.

Aqueous phase of the compositions of the present invention comprises at least one cationic surfactant at a concentration of 0.05 to 5%, preferably 0.1 to 3%, more preferably 0.2 to 2.5% by weight calculated to the content of the aqueous phase. Suitable cationic surfactants are according to the general formula 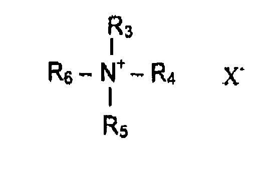 where R₃ is a saturated or unsaturated, branched or non-branched alkyl chain with 8-22C atoms or

R₇ CO NH (CH₂)ₙ

where R₇ is saturated or unsaturated, branched or non-branched alkyl chain with 7-21C atoms and n has typical value of 0 - 4 or

R₈ CO O (CH₂)ₙ

where R₈ is saturated or unsaturated, branched or non-branched alkyl chain with 7-21C atoms and n has typical value of 0 - 4, and
R₄ is a hydrogen, saturated or unsaturated, branched or non-branched alkyl chain with 1-22 C atoms or

R₇ CO NH (CH₂)ₙ

where R₇ is saturated or unsaturated, branched or non-branched alkyl chain with 7-21 C atoms and n has typical value of 0 - 4 or

R₈ CO O (CH₂)ₙ

where R₈ is saturated or unsaturated, branched or non-branched alkyl chain with 7-21 C atoms and n has typical value of 0 - 4, and R₅ and R₆ are independent from each other H or lower alkyl chain with 1 to 4 carbon atoms, and X is chloride, bromide or methosulfate.

Suitable cationic surfactants are particularly cetyl trimethly ammonium chloride, steartrimonium chloride, behentrimoinium chloride, stearamidopropyl trimonuim chloride, behenamidopropylethyldimonium ethosulfate, behenamidopropyltrimonium methosulfate, cocamidopropyltrimonium chloride, cocotrimonim chloride, palmitamidopropyltrimonum chloride, dipalmitoyltrimonium chloride, di-C12-C15 alkyldimoniumchloride, distearyldimonium chloride, dipalmitoylethylhydroxyethylmonium chloride, dioleoylethyl dimethyl ammonium methosulfate, dioleoylethyl hydroxyethylmoniun methosulfate, dilinolamidopropyldimonium chloride, dioleylethyl hydroxyethylmonium chloride and dipalmitoylethyldimonium chloride.

The pH of the aqueous phase varies from 3 to 7, particularly 4.5 to 6. For adjusting the pH of the said conditioner compositions, following ingredients can be used; Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, pyruvic acid, salicylic acid or acetic acid or inorganic acids such as hydrochloric acid, phosphoric acid, sulphuric acid, nitric acid. The pH of the conditioner composition can also be adjusted to the required pH by using alkaline solution such as sodium hydroxide, potassium hydroxide, ammonium hydroxide or their salts with those acids mentioned above.

Compositions of the present invention can comprise UV filters for protection of hair from environmental influences. It should be noted that oil soluble UV filters are contained in the oil phase and water soluble ones are added to the aqueous phase although partitioning the UV filter between two phases is not excluded. The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxybenzophenone, 2-hydroxy-4-methoxybenzophenone and its 5-sulfonic acid or the sodium salt thereof, 2,2'-dihydroxy-4,4'-dimethoxybenzaphenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzaphenone, 2-hydroxy-4-methoxy-4'-methylbenzophenone, 3-benzyl-idenecampher, 3-(4'-sutfo)-benzyl-idenebornane-2-ono and the salts thereof, 3-(4'-methyl benzylidene)-DL-campher, 2-ethylhexyl.-2-cyano-3,8-diphenyl acrylate also known as Octocrylene and/or polysilicone-15.

The preferred amount of the UV-absorber ranges from about 0.01 % to 2.5%, more preferably from 0,05 % to 1 % by weight, calculated to the total composition.

The moisturising agents may be included into the aqueous phase of the compositions. Suitable ones are panthenol, polyols, such as glycerol, polyethylene glycols with molecular weight 200 to 20,000. The moisturising ingredients can be included in the aqueous phase of the compositions at a concentration range of 0.01 - 2.5% by weight calculated to the content of the aqueous phase.

Aqueous phase may comprise 0.1 to 20%, preferably 0.5 to 15%, more preferably 0.5 to 10 % by weight calculated to the content of aqueous phase at least one fixing polymer. The composition can also comprise mixture of more than one fixing polymer. In selecting fixing polymers attention should be paid to the solubility of the polymer in aqueous phase as described above and also in the total composition
wherein oil and aqueous phases are present together and mixed to become homogeneous composition by mixing.

Suitable polymers are non-ionic, cationic and amphoteric polymers. Anionic polymers may also be used so far if no incompatibility is observed in the presence of cationic surfactant. One of the important point in selecting fixing polymer is that the polymer should not thicken the aqueous phase. In other words the viscosity of the aqueous phase should remain low i.e. below 100 mPa.s. preferably 50 mPa.s.

Suitable non-ionic polymers are polyvinylpyrrolidone, polyvinylpyrrolidone/vinylacetate copolymer and polyvinylpyrrolidone/vinylacetate/vinylpropionate copolymer. Preferred is polyvinylpyrrolidone/vinylacetate copolymer. In addition to these fixing polymers derivatives of natural polymers such as hydroxyl ethylcellulose may also be used in combination with one of the fixing polymers mentioned above.

Suitable cationic polymers include Polyquaternium-4, Polyquaternium-11, Polyquaternium-16, Polyquaternium-18, Polyquaternium-24, Polyquaternium-28, Polyquaternium-46, polyvinylpyrrolidone/dimethylaminoethyl methacrylate, and cationically derivatized natural polymers. Preferred are Polyquaternium-4, Polyquaternium-11 and Polyquaternium-16.

Suitable amphoteric polymers are those available from the company National Starch under the trade name Amphomer such as Octylacrylamide/Acrylates/Butylaminoethyl methacrylate copolymer, those available under the trade name Diaformer (methacryloylethylbetaine/methacrylates copolymer). Preferred are the ones available under the trade name Amphomer.

In another preferred from of the invention, aqueous phase of the composition comprises combination of non-ionic and cationic fixing polymers.

As a fixing polymer silicone containing polymers either grafted or block copolymer are also useful. Suitable and the preferred one is Polysilicone-9.

The aqueous and oil phases of the compositions of the present invention may be coloured. The dyestuff suitable for product colouring purposes are all useful for this purpose. It should be noted that for colouring aqueous phase water soluble dyes should be used and for colouring oil phase oil soluble dyes are suitable. The nature of the dyestuff is actually not important but preferred are non substantive dyes, i.e. not remaining on hair after washing with water or shampooing.

The additional component phase c is preferably in the emulsion form and it comprises at least one fatty alcohol with linear of branched alkyl chain. Suitable ones are fatty alcohols having 12 to 22 C atoms in its alkyl chain. Examples are myristyl alcohol, cetyl alcohol, stearyl alcohol, behenyl alcohol and their mixtures. Preferred are cetyl, stearyl and behenyl alcohol and their mixtures i.e. cetearyl alcohol. Fatty alcohols may be included into the compositions of the present invention at a concentration of 0.1 to 20%, preferably 0.5 to 15% and more preferably 1 to 10% by weight calculated to total composition.

Emulsions should also comprise at least one emulsifier. It should be noted that quaternary ammonium compounds with single alkyl chain mentioned above are preferred as emulsifiers as well.

In addition to the cationic surfactants with single alkyl chain, additional emulsifier can be incorporated into the compositions. These additional emulsifiers are surface active substances such as non-ionic, amphoteric or zwitterionic and anionic compounds. Because of the compatibility issues of the anionic surfactants and cationic surfactants mentioned above, anionic surfactants are less suitable and therefore their compatibility should carefully be examined. Otherwise, the compositions of the present invention preferably should substantially be free of anionic surfactants, Preferred emulsifiers are cationic, non-ionic, amphoteric or zwitterionic surfactants, The most preferred additional emulsifiers are non-ionic surfactants.

### Suitable non-ionic surfactants are alkyl polyglucosides of the general formula

R₇-O- (R₈O)ₙ O- Zx

wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono-and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates, Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e,g., "Laureth-16":

The average degree of ethoxylation thereby ranges between about 2.5 and about 25, preferably about 10 and about 20.

Among the non-ionic surfactants mentioned above fatty alcohol ethoxylates are the most preferred ones. Above mentioned non-ionic surfactants can also be used as mixture of one category such as several ethoxylated fatty alcohols or several categories such as mixture of alkyl polyglucoside and ethoxylated fatty alcohol.

Emulsifier content of the compositions of phase c according to present invention is in the range of 0.05 to 10%, preferably 0.1 to 7.5% and more preferably 0.25 to 5% by weight calculated to total composition,

Compositions of the present invention in phase c can comprise additional hair conditioning compounds such as oils, cationic polymers, non-ionic substances. Oils as conditioners according to the present invention are selected from silicone oils either volatile or non-volatile, natural and synthetic oils. Among silicone oils those can be added to the compositions include dimethicone, dimethiconol, polydimethylsiloxane and DC fluid ranges from Dow Corning, cyclosiloxanes such as DC 245 and arylated silicones such as phenyl methicone, phenyl trimethicone, diphenyl dimethicone, diphenylsiloxy phenyl trimethicone, tetramethyl tetraphenyl trisiloxane, triphenyl trimethicone, tetramethly tetraphenyl trisiloxane and trimethyl pentaphenyl trisiloxane. Synthetic oils include mineral oil such as paraffin oil and petrolatum.

Natural oils suitable are such as olive oil, almond oil, avocado oil, weizenkeirn oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof. Lipophilic oily compounds such as fatty acid esters are as well suitable for the composition of the present invention. Those are such as isopropyl myristate, palmitate, stearate and isostearate, oleyl oleate, isocetyl stearate, hexyl laurate, dibutyl adipate, dioctyl adipate, myristyl myristate, oleyl erucate, cetyl palmitate, etc.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₉ CO (OCH₂CH₂)ₙ OH

R₉ CO (OCH₂CH₂)ₙ O OC R₁₀

where R₉ and R₁₀ are independent from each other saturated, unsaturated or branched or non-branched alkyl chain with 7 to 21 C atoms and n is typically 2 - 100. Typical concentration range for any of the additional conditioners mentioned above other than cationic conditioning compounds can be in the range of 0.01 to 15% by weight, preferably 0.05 -10% by weight, more preferably 0.1-5% by weight calculated to the total composition.
Composition of the present invention can comprise in phase c cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has especially been found suitable those cationic polymers known with their CTFA category name Polyquaternium, Typical examples of those Polyquaternium 4, Polyquaternium 6, Polyquaternium 7, Polyquaternium 10, Polyquaternium 11, Polyquaternium 16. Polyquaternium 22, Polyquaternium 24, Polyquaternium 28, Polyquaternium 30, Polyquaternium 37, Polyquaternium 36, Polyquaternium 46, Polyquaternium 67 and Polyquaternium 72.

As well those polymers known with their CTFA category name Quaternium are suitable. Those are for example Quaternium-8, Quaternium-14, Quaternium-15, Quatemium-18, Quaternium-22, Quafiernium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quatemium-82. Quaternium-83 and Quaternium-84.

In this context, reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quaternized products of graft polymers from organopolysiloxanes and polyethyl oxazolines described in EP-A 524 612 and EP-A 640 643.

The above mentioned cationic polymers can also be present in the aqueous phase, phase b if their water solubility makes it possible.

The compositions according to the present invention can also comprise further agents, such as protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin".

Additional natural plant extracts can as well form part of the composition of the present invention in any of the phases a, b and c. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, green tea, blue lotus flower, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Plant extracts may also be oil soluble fractions and in such a case they may also pbe comprised in phase a, oil phase.

Suitable trade products are, for example, the various "Extrapone" products and "Herbasol^{R} ". Extracts and the preparation thereof are also described in "Hagars Handbuch der pharmazeutischen Praxis", 4^{th} Ed.

The compositions may contain organic solvents such as ethanol, propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the composition should not exceed 10%, preferably between 1 and 5% by weight, calculated to total composition. It should be noted that penetration enhancers are useful for both cleansing and after-shampoo conditioning preparations. It is obvious that the concentration in the cleansing compositions is usually lower than in the conditioning preparations.

Fragrance, chelating agent, preservatives and other conventional cosmetic ingredients can be included at their usual concentrations either into the oil or aqueous phases depending on their solubility.

The composition of the present invention is preferably transparent, i.e. both oil and aqueous phases, phases a and b as mentioned above, are transparent as judged by a naked eye in a transparent glass or PET bottle with a thickness of up to 3 cm. The additional component is preferably non transparent. The composition formed by mixing all three components is also preferably non transparent, Composition of the present invention is leave-in composition, i.e. not rinsed off from hair after application. Accordingly, in a process composition of the present invention is applied to the shampooed and/or wetted hair and hair is dried. The compositions of the present invention are preferably applied onto hair as a lotion directly from the vessel wherein they are mixed into a homogeneous composition.

Mixing ratio of the three phases of the present invention should be being oil phase : aqueous phase : third composition in the range of 0.1 : 1 : 0.1 to 1:1:1, preferably 0.2 : 1 : 0.2 to 0.8 : 1 : 0.8. more preferably 0.3 :1 : 0.3 to 0.6 : 1 : 0.6. The mixing rato may as well be 0.2 : 1 : 0.6, or 0.5:1:0.2 etc.

Following examples are to illustrate the invention but not to limit,

### Example 1

**Oil phase**

| | % by weight |
|---|---|
| Trisiloxane | 94.8 |
| Propylene glycol dibenzoate | 5.0 |
| Fragrance | 0.2 |

**Aqueous phase**

| | % by weight |
|---|---|
| VPNA copolymer | 3.5 |
| Steartrimoniumchloride | 0.2 |
| Di-C₁₂-C₁₅ alkyldimonium chloride | 0.1 |
| Ethanol | 23.0 |
| Citric acid/Ammonium hydroxide | q.s. to pH 6.0 |
| Preservative | q.s. |
| Water | to 100 |

**Third phase**

| | % by weight |
|---|---|
| Cetearyl alcohol | 5.0 |
| Cetrimonium chloride | 2.0 |
| Benzyl alcohol | 5.0 |
| Trimethyl phentaphenyl trisiloxane | 0.5 |
| Citric acid/ ammonium hydroxide | q.s. to pH 6.0 |
| Preservative | q.s. |
| Water | to 100. |

From the above composition oil and aqueous phase are clear and when stored together in a vessel there is a third phase formation at the bottom of the vessel which does not affect the performance or further stability. The additional component is a non-tranparent emulsion.

The compositions were confectioned in a single vessel including an upper vessel allowing separate storage of the additional third phase in the same unit.

The composition is packed with the following weight ratio of oil phase : aqueous phase : third phase: 0.3 : 1 : 0.2.

The composition was mixed to homogeneity by shaking in the same vessel and applied onto freshly washed hair and without rinsing off hair was dried. The hair has excellent shine, combability and volume and body and hair felt very soft upon touching.

Exclusion of Propylene glycol dibenzoate from the oil phase resulted in significant loss of shine and hair felt slightly less soft.

### Example 2

**Oil phase**

| | % by weight |
|---|---|
| Trisiloxane | 98.6 |
| Propylene glycol dibenzoate | 1.0 |
| Benzophenone-3 | 0.2 |
| Fragrance | 0.2 |

**Aqueous phase**

| | % by weight |
|---|---|
| Polyqauternium-16 | 0.8 |
| Benzophenone-4 | 0.1 |
| Ethanol | 20.0 |
| Lactic acid/Ammonium hydroxide | q.s. to pH 5.0 |
| Water | to 100 |

**Third phase**

| | % by weight |
|---|---|
| Behenyl alcohol | 3.0 |
| Behenrimonium chloride | 1.0 |
| Propylene glycol | 5.0 |
| Trimethyl phentaphenyl trisiloxane | 0.5 |
| Citric acid/ ammonium hydroxide | q.s. to pH 6.0 |
| Preservative | q.s. |
| Fragrance | 0.1 |
| Water | to 100. |

From the above composition oil and aqueous phase are clear and when stored together in a vessel there is a third phase formation at the bottom of the vessel which does not affect the performance or further stability. The additional component is a non-tranparent emulsion.

The compositions were confectioned in a single vessel including an upper vessel allowing separate storage of the additional third phase in the same unit.

The composition is packed with the following weight ratio of oil phase : aqueous phase : third phase: 0.2 : 1 : 0.4.

The composition was mixed to homogeneity by shaking in the same vessel and applied onto freshly washed hair and without rinsing off hair was dried. The hair has excellent shine, combability and volume and body and hair felt very soft upon touching.

Exclusion of Propylene glycol dibenzoate from the oil phase resulted in significant loss of shine and hair felt slightly less soft.

### Example 3

**Oil phase**

| | % by weight |
|---|---|
| Dimethicone 1 cst | 97.5 |
| Propylene glycol dibenzoate | 2.0 |
| Ethyl hexyl methoxy cinnamate | 0.3 |
| Fragrance | 0.2 |

**Aqueous phase**

| | % by weight |
|---|---|
| VPNA copolymer | 3.0 |
| Polysilicone-9 | 0.5 |
| Cetrimonium chloride | 0.15 |
| Dioleylethyl hydroxyethylmonium chloride | 0.15 |
| Benzophenone-4 | 0.10 |
| Ethanol | 25.0 |
| Lactic acid/Ammonium hydroxide | q.s. to pH 6.0 |
| Dyestuff Cl 19140 and Cl 42053 | q,s, |
| Water | to 100 |

**Third phase**

| | % by weight |
|---|---|
| Behenyl alcohol | 3.0 |
| Behenrimonium chloride | 1.0 |
| Propylene glycol | 5.0 |
| Trimethyl phentaphenyl trisiloxane | 0.5 |
| Citric acid/ ammonium hydroxide | q.s. to pH 6.0 |
| Preservative | q.s. |
| Fragrance | 0,1 |
| Water | to 100. |

From the above composition oil and aqueous phase are clear and when stored together in a vessel there is a third phase formation at the bottom of the vessel which does not affect the performance or further stability. The additional component is a non-transparent emulsion.

The compositions were confectioned in a single vessel including an upper vessel allowing separate storage of the additional third phase in the same unit.

The composition is packed with the following weight ratio of oil phase : aqueous phase : third phase: 0.4 1 : 0.3.

The composition was mixed to homogeneity by shaking in the same vessel and applied onto freshly washed hair and without rinsing off hair was dried. The hair has excellent shine, combability and volume and body and hair felt very soft upon touching.

Exclusion of Propylene glycol dibenzoate from the oil phase resulted in significant loss of shine and hair felt slightly less soft.

Additionally, fly aways were significantly reduced, hair was easily managable and had excellent elasticity

## Claims

1. Multiphase composition for hair **characterised in that** it comprises
a- 5 to 30%, by weight oil phase, calculated to total composition, comprising at least one volatile oil,
b- 50 to 75%, by weight, aqueous phase, calculated to total composition, and
c- 5 to 45% by weight calculated to total composition an additional composition preferably an emulsion comprising at least one oil and at least one emulsifier in an aqueous medium,
wherein a compound selected from compounds according to general formula 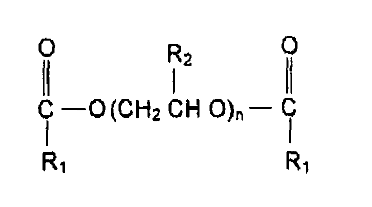 wherein R₁ is a phenyl and R₂ is H or a C1 to C4 alkyl and n is a number between 1 and 5 and compounds of dipropylene glycol dibenzoate and trimethylpentanediyl dibenzoate is included into one of the phases except aqueous phase at a concentration of 0.1 to 20% by weight calculated to total composition.

2. Composition according to claim 1 **characterised in that** compounds according to formula are diethyleneglycol dibenzoate, propylene glycol dibenzoate and triethylene glycol dibenzoate.

3. Composition according to claims 1 and 2 **characterised in that** it comprises at least one volatile oil at a concentration of 70 to 99.9% by weight calculated to total content of oil phase (phase a).

4. Composition according to claim 3 **characterised in that** at least one volatile oil is selected from dimethicones with viscosity of 5 mm²/s at 20°C and cyclomethicones.

5. Composition according to any of the preceding claims **characterised in that** aqueous phase (phase b) comprises at least one organic solvent at a concentration of 10 to 40% by weight calculated to total content of aqueous phase.

6. Composition according to any of the preceding claims **characterised in that** aqueous phase (phase b) comprises at least one cationic surfactant according to general formula 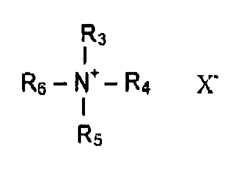 where R₃ is a saturated or unsaturated, branched or non-branched alkyl chain with 8-22 C atoms or
R₇ CO NH (CH₂)ₙ
where R₇ is saturated or unsaturated, branched or non-branched alkyl chain with 7-21 C atoms and n has typical value of 0 - 4 or
R₈ CO O (CH₂)ₙ
where R₈ is saturated or unsaturated, branched or non-branched alkyl chain with 7-21 C atoms and n has typical value of 0 - 4, and
R₄ is a hydrogen, saturated or unsaturated, branched or non-branched alkyl chain with 1-22 C atoms or
R₇ CO NH (CH₂)ₙ
where R₇ is saturated or unsaturated, branched or non-branched alkyl chain with 7-21 C atoms and n has typical value of 0 - 4 or
R₈ CO O (CH₂)ₙ
where R₈ is saturated or unsaturated, branched or non-branched alkyl chain with 7-21 C atoms and n has typical value of 0 - 4,
and R₅ and R₆ are independent from each other H or lower alkyl chain with 1 to 4 carbon atoms, and X is chloride, bromide or methosulfate.

7. Composition according to any of the preceding claims **characterised in that** it comprises at least one UV filter in any of the phases a, b and/or c.

8. Composition according to any of the preceding claims **characterised in that** It comprises at least one natural plant extract in any of the phases a, b and/or C.

9. Composition according to any of the preceding claims **characterised in that** it comprises at least one fixing polymer in aqueous phase (phase b) at a concentration of 0.1 to 20% by weight calculated to total content of aqueous phase.

10. Composition according to an of the preceding claims **characterised in that** it comprises at least one fatty alcohol and at least one emulsifier in the third phase (phase c).

11. Composition according to any of the preceding claims **characterised in that** the three phases are mixed at a weight ratio 0.1 : 1 : 0.1 to 1:1:1 of oil phase (phase a) : aqueous phase (phase b) : third pahse (phase c).

12. Use of composition according to any of the preceding claims for enhacing shine, combability, volume and body and manageability of hair.

13. Process for conditioning hair **characterised in that** a composition according to claims 1 to 11 is mixed to homogeneity and applied onto cleansed or wetted hair.

14. Product for conditioning hair **characterised in that** the phases a an b according to claims 1 to 11 are stored in the same vessel as optically separated two or more phases and the additional composition c according to claims 1 to 11 is kept separate until use in a different packaging and all phases are mixed together into a homogeneous composition in another vessel prior to application onto hair.

15. Product for conditioning hair **characterised in that** the phases a an b according to claims 1 to 11 are stored in the same vessel as optically separated two or more phases and additional composition c according to claims 1 to 11 is kept separate until use in the same packaging in a separate compartment and all phases are mixed together into a homogeneous composition in the same vessel prior to application onto hair.
